Europäisches Patentamt

European Patent Office

Office européen des brevets

(19)

(11) **EP 0 752 102 B1**

(12) **FASCICULE DE BREVET EUROPEEN**

(45) Date de publication et mention
de la délivrance du brevet:
**15.11.2000 Bulletin 2000/46**

(21) Numéro de dépôt: **96901840.7**

(22) Date de dépôt: **23.01.1996**

(51) Int Cl.7: **G01N 33/535**, G01N 33/58

(86) Numéro de dépôt international:
**PCT/FR96/00113**

(87) Numéro de publication internationale:
**WO 96/23226 (01.08.1996 Gazette 1996/35)**

(54) **CONJUGUE IMMUNOENZYMATIQUE, SON PROCEDE DE PREPARATION ET SES APPLICATIONS**

IMMUNOENZYMATISCHES KONJUGAT, HERSTELLUNGSVERFAHREN DAFÜR UND VERWENDUNGEN

IMMUNOENZYMATIC CONJUGATE, PREPARATION METHOD THEREFOR AND USES THEREOF

(84) Etats contractants désignés:
**AT BE CH DE DK ES FR GB GR IE IT LI LU MC NL PT SE**

(30) Priorité: **23.01.1995 FR 9500735**
**18.05.1995 FR 9505939**

(43) Date de publication de la demande:
**08.01.1997 Bulletin 1997/02**

(73) Titulaire: **PASTEUR SANOFI DIAGNOSTICS**
**92430 Marnes La Coquette (FR)**

(72) Inventeurs:
• **CUCUROU, Christophe**
**F-92210 Saint-Cloud (FR)**

• **COGNET, Gilles**
**F-91330 Yerres (FR)**
• **GADELLE, Stéphane**
**F-91570 Bièvres (FR)**
• **LE SAGER, Carine**
**F-78400 Chatou (FR)**

(74) Mandataire: **Le Guen, Gérard et al**
**CABINET LAVOIX**
**2, place d'Estienne d'Orves**
**75441 Paris Cédex 09 (FR)**

(56) Documents cités:
| | |
|---|---|
| **EP-A- 0 175 560** | **EP-A- 0 209 155** |
| **EP-A- 0 430 510** | **WO-A-92/10507** |
| **DE-A- 4 237 479** | **US-A- 4 693 969** |

**Description**

La présente invention concerne un conjugué immunoenzymatique constitué de substance(s) à activité immunologique et d'enzyme(s) de marquage glycolysée(s) sous forme de copolymère. L'invention concerne également le procédé de préparation du conjugué selon l'invention et l'utilisation dudit conjugué pour le diagnostic.

On connaît de l'art antérieur différents conjugués et procédés de préparation de conjugués immunoenzymatiques. Ces conjugués immunoenzymatiques sont constitués généralement d'une substance à activité immunologique et d'une enzyme de marquage qui peut être glycosylée. Les procédés de préparation de ces conjugués sont variés et il existe particulièrement des procédés tels que ceux utilisant des techniques de couplage au périodate, lesquels ont donné lieu à des techniques plus perfectionnées utilisant des agents homo ou hétérobifonctionnels. Ces procédés ont pour but d'améliorer le couplage de l'enzyme à la substance à activité immunologique afin d'obtenir des conjugués qui permettent d'augmenter la spécificité et/ou la sensibilité des résultats de diagnostic.

Le procédé de couplage de Nakane (The Journal of Histochemistry and Cytochemistry, vol.22 n°.12, pp. 1084-1091, 1974) décrit, après l'oxydation des carbohydrates de l'enzyme au périodate, le couplage direct de la protéine par l'intermédiaire de ses fonctions amines libres. Le brevet EP 209155 décrit un procédé permettant le couplage du réactif immunologique sur la partie protéique de l'enzyme. Selon ce procédé, l'enzyme est soumise à une oxydation à l'aide de l'acide périodique avant ou après son couplage avec le réactif immunologique. Le produit d'oxydation ainsi obtenu est ensuite réduit avec du borohydrure de sodium. En ce qui concerne le couplage, ceci est réalisé à l'aide de réactifs classiques, tels que la glutaraldéhyde, ou de dérivés de succinimide. Ce procédé ne met en oeuvre aucune étape de copolymérisation. Les conjugués issus de ce procédé permettent d'éliminer les résultats faux positifs provenant de sérums dits "à problèmes".

De même, la demande de brevet EP 601318 décrit un procédé de couplage du réactif immunologique sur l'enzyme par l'intermédiaire d'une diamine puis d'un réactif hétérobifonctionnel (dérivé du succinimide), la diamine ayant au préalable réagi sur la partie oxydée du carbohydrate de l'enzyme. On obtient de la sorte un conjugué non copolymérisé, dont la liaison enzyme-réactif immunologique se trouve sur la partie carbohydrate de l'enzyme. En effet, selon ce procédé de couplage, les groupements carbohydrates portés par la protéine sont mis en oeuvre pour greffer directement la protéine à l'anticorps.

Plus récemment, la demande de brevet EP 560912 et le brevet US 5,191,066 décrivent un procédé de préparation d'immunoconjugués (anticorps-phosphatase alcaline) utilisant la partie carbohydrate des protéines, ce qui permet de préserver le site de reconnaissance immunologique de l'anticorps.

On connaît aussi certains procédés permettant d'obtenir des conjugués immunoenzymatiques sous forme de copolymères.

Le brevet US 4,693,969 décrit un réactif pour immunoessais de type sandwich et son procédé de préparation. Le réactif pour immunoessais, comportant un conjugué immunoenzymatique sous forme polymérisée, permet d'augmenter la sensibilité des dosages de substances présentes à de faibles concentrations dans des échantillons biologiques, telles que les hormones. Ce réactif est obtenu en synthétisant, dans un premier temps, des immuno-conjugués anticorps/enzyme sous forme de monomère, à l'aide d'un agent de couplage (m-maléimidobenzoyl N-hydroxysuccinimide ester). Dans un deuxième temps, les monomères sont soumis à une réaction de polymérisation avec du glutaraldéhyde ou du carbodiimide.

La demande EP 430510 décrit un polymère à base d'unités "détectables" reliées entre elles par un agent de couplage. Ces unités "détectables", soit ont une activité antigénique, soit font partie d'un système fluorescent, chimioluminescent, chromogénique ou enzymatique. Les unités "détectables" sont reliées entre elles par un agent de couplage hydrophile qui est un dérivé de 1,4-di(aminoalkyl) pipérazine. Pour obtenir ces polymères, les unités "détectables" sont modifiées chimiquement de manière à comporter soit deux groupes carboxyles, soit un groupement carboxyle et un groupe amino ou thiocyanate. La polymérisation avec les dérivés de 1,4-di(aminoalkyl) pipérazine est effectuée en mettant en oeuvre des méthodes classiques utilisées, soit dans la chimie de préparation de peptides à partir des acides aminés, soit dans la chimie de synthèse de polymères (utilisation de réactifs de condensation de peptides ou de dialdéhydes).

La demande EP 175560 décrit un procédé d'obtention d'un conjugué constitué d'une enzyme polymérisée et d'un anticorps. Selon ce procédé, on obtient dans un premier temps, par couplage covalent d'au moins deux molécules d'enzyme, par l'intermédiaire de leurs fonction thiols ou amines libres, une enzyme prépolymérisée. Cette enzyme prépolymérisée est ensuite couplée par des liaisons covalentes à un anticorps ou un fragment d'anticorps.

A l'heure actuelle, il est nécessaire pour certaines pathologies de détecter très précocement la présence d'un antigène ou d'un anticorps spécifique. De plus, certains antigènes sont parfois masqués à la détection et seules quelques infimes quantités d'anticorps spécifiques de l'antigène apparaissent dans le sérum du patient atteint d'une telle infection.

Les conjugués connus de l'art antérieur ne permettent pas une détection suffisamment sensible des antigènes ou anticorps présents dans le sérum de patients atteints d'infection qui n'induit pas de réaction immunologique importante.

La présente demande se propose d'apporter une solution à ce problème de manque de sensibilité par l'utilisation des conjugués immunoenzymatiques selon l'invention.

L'invention a pour objet des conjugués immunoenzymatiques, lesquels conjugués sont constitués d'enzymes de marquage glycosylées sous forme de copolymère et de substances à activité immunologique, et l'utilisation de ces conjugués dans un dosage immunologique.

L'invention concerne également le procédé de préparation des conjugués immunoenzymatiques copolymérisés selon l'invention, ainsi que l'utilisation desdits conjugués pour la détermination immunologique.

L'invention concerne encore des trousses pour le diagnostic comprenant les conjugués selon l'invention.

Les termes "carbohydrate" ou "glycosyle", associés à l'enzyme, seront utilisés par la suite pour signifier que l'enzyme possède un ou plusieurs groupements glucidiques liés sur sa partie protéique.

Les conjugués selon l'invention permettent d'obtenir des résultats beaucoup plus sensibles que ceux obtenus par les conjugués immunoenzymatiques déjà connus de l'art antérieur. L'invention apporte donc une amélioration significative dans le domaine du diagnostic, et spécialement pour le diagnostic des pathologies ayant une origine virale puisque ces dernières sont souvent de détection, donc de diagnostic, difficile au début de l'infection.

La présente invention concerne plus particulièrement des conjugués immunoenzymatiques constitués:

- de molécules d'enzyme de marquage copolymérisées entre elles par l'intermédiaire de leurs groupes glucidiques préalablement oxydés, de manière à former un copolymère d'enzyme,
- et d'au moins une substance à activité immunologique conjuguée aux molécules d'enzyme de marquage copolymérisée par l'intermédiaire de fonctions amines libres du copolymère d'enzyme.

Les conjugués selon l'invention sont constitués d'enzymes de marquage, possédant une partie carbohydrate, telles que la peroxydase de raifort (POD), la phosphatase alcaline (PAL), la galactosidase, la glucose-oxydase et la fructose-oxydase. On obtient ainsi, par le procédé selon l'invention, des conjugués constitués de copolymères constitués desdites enzymes citées, copolymères selon l'enzyme choisie.

Selon l'invention, le copolymère d'enzyme est obtenu à partir d'enzyme de marquage et de diamine(s), ou d'enzyme de marquage et de réactifs hétérobifonctionnels différents, liés entre eux.

Selon une variante avantageuse, l'invention concerne un conjugué dont le copolymère d'enzyme est obtenu à partir d'une diamine choisie parmi une diamine aliphatique (à chaîne linéaire ou ramifiée ou cyclisée) ou une diamine aromatique, lesquelles amines comprennent de 2 à 12 atomes de carbone. On utilise de manière préférentielle la phénylène-1,4-diamine.

Selon une autre variante avantageuse, l'invention concerne un conjugué dont le copolymère d'enzyme contient deux réactifs hétérobifonctionnels différents, liés entre eux, qui sont respectivement choisis parmi la 2-mercaptoéthylamine ou la 3-(2-pyridyl-dithio) propionyl hydrazide, et la 4-(N-maléimidométhyl) cyclohexane-1-carboxyl-hydrazide ou le 4-(4-N-maléimido-phényl) butyryl hydrazide.

Plus particulièrement, l'invention concerne un conjugué dont le copolymère d'enzyme comprend des proportions d'enzymes et de diamines ou de réactifs hétérobifonctionnels respectivement de 1/1-10 équivalents molaires, de préférence 1/4-6 équivalents molaires.

De façon préférentielle, l'invention concerne un conjugué dont le copolymère d'enzymes comprend n molécules d'enzyme, n étant un nombre entier compris entre 3 et 100, de préférence un nombre entier compris entre 5 et 50.

Selon une variante avantageuse, l'invention concerne un conjugué dont l'enzyme copolymérisée est la peroxydase de raifort ou la phosphatase alcaline.

Le conjugué immunoenzymatique de l'invention est un conjugué dont le copolymère d'enzyme(s) est couplé à au moins une substance à activité immunologique par l'intermédiaire d'un réactif homo ou héterobifonctionnel, qui réagit avec une fonction amine du copolymère d'enzyme.

Plus particulièrement, l'invention concerne un conjugué dont les proportions molaires respectives du copolymère d'enzymes et de substance(s) à activité immunologique sont de 10/1 à 1/10 (unité enzymatique/unité de substance à activité immunologique), de préférence 3/1 à 1/3, et de préférence encore 1/1.

Les substances à activité immunologique couplées aux copolymères d'enzymes peuvent être des protéines naturelles ou recombinantes, des anticorps mono ou polyclonaux, recombinants ou non, ou des peptides tels que des peptides dérivés des virus tels que les virus VIH (1 ou 2), VHC et VHB, recombinants ou non.

Selon une variante préférée, l'invention concerne un conjugué dont la substance à activité immunologique est un peptide VIH1, un peptide VIH2, un peptide VHC, une protéine recombinante VHC, un anticorps-anti-VIH1 ou un anticorps anti AgHBs.

Les réactifs de couplage homo ou hétérobifonctionnels utilisés dans l'étape de couplage du copolymère d'enzyme avec la substance à activité immunologique peuvent être, par exemple, le bis(sulfosuccinimidyl) subérate (BS³), le sulfosuccinimidyl-4-(N-maléimidométhyl) cyclo-hexane-1-carboxylate (sulfo-SMCC) ou le N-succinimidyl-3-(2-pyridyldithio) propionate (SPDP).

L'invention a également pour objet un procédé de préparation du conjugué selon l'invention, caractérisé en ce que :

a. on copolymérise les molécules d'enzyme de marquage par l'intermédiaire de leurs carbohydrates préalablement oxydés,

b. puis on effectue le couplage du copolymère d'enzyme avec au moins une substance à activité immunologique.

Pour l'oxydation du carbohydrate d'enzyme, on utilise des réactifs permettant d'obtenir des groupes aldéhydes. On met en oeuvre, par exemple, une oxydation par le periodate ou toute autre oxydation équivalente permettant d'obtenir des groupes aldéhydes.

Dans un premier mode de réalisation, dans l'étape de copolymérisation du procédé de préparation de conjugués selon l'invention, on fait réagir les molécules d'enzyme avec une diamine.

Dans un deuxième mode de réalisation, dans l'étape de copolymérisation du procédé de préparation de conjugués selon l'invention, on fait réagir séparément dans une première étape les molécules d'enzyme(s) avec deux réactifs hétérobifonctionnels différents, puis dans une deuxième étape on fait réagir entre eux les produits de la réaction.

Selon une variante avantageuse, l'invention concerne un procédé de préparation d'un conjugué immunoenzymatique copolymérisé dans lequel après la copolymérisation des molécules d'enzyme(s) de marquage, lorsqu'on utilise une diamine, on effectue une réduction avec un agent réducteur tel que le borohydrure de sodium ou le cyanoborohydrure de sodium, ce qui a pour effet d'arrêter la copolymérisation.

Selon une autre variante avantageuse, l'invention concerne un procédé de préparation d'un conjugué immunoenzymatique copolymérisé dans lequel après la copolymérisation de molécules d'enzyme(s) de marquage, lorsqu'on utilise des réactifs hétérobifonctionnels différents, on effectue une réaction avec des agents bloquant les fonctions libres des réactifs hétéro-bifonctionnels qui n'ont pas réagi. On peut, selon le cas, soit utiliser les deux agents bloquant les fonctions libres des deux réactifs utilisés, soit utiliser un des deux agents bloquant les fonctions libres des réactifs employés.

De façon préférentielle, l'invention concerne un procédé de préparation d'un conjugué immunoenzymatique copolymérisé selon lequel dans l'étape du couplage du copolymère d'enzyme avec les substances à activité immunologique, la concentration du réactif de couplage homo ou hétérobifonctionnel est en excès par rapport à la concentration du copolymère d'enzyme.

Le procédé de préparation selon l'invention est décrit de façon plus détaillée ci-dessous pour une copolymérisation effectuée en une étape:

L'enzyme choisie pour effectuer la préparation du conjugué est, préalablement à la réaction de copolymérisation, traitée avec un réactif oxydatif des groupements carbohydrates, tel que le périodate (plusieurs centaines de moles/moles d'enzyme) à un pH compris entre 4 et 8. On élimine ensuite le périodate en excès par dialyse de la solution d'enzyme oxydée.

On ajoute alors une diamine aliphatique (linéaire, ramifiée ou cyclisée) ou aromatique comprenant de 2 à 12 atomes de carbone, de préférence la phénylène-1,4-diamine, à une concentration de 1/1-10 équivalents molaires (unité enzymatique/unité de diamine), de préférence de 1/4-6 équivalents molaires, en un milieu dont le pH est compris entre 7 et 9,5.

On contrôle la réaction de copolymérisation par analyse chromatographique de filtration sur gel pour obtenir des copolymères d'enzyme qui comprennent n motifs d'enzymes, n étant un nombre entier de 3 à 100, de préférence un nombre entier de 5 à 50.

On arrête alors la réaction de copolymérisation en ajoutant en excès un réducteur des carbohydrates oxydés, qui n'ont pas réagi pendant la réaction de copolymérisation, tel que le borohydrure de sodium ou le cyanoborohydrure de sodium.

On purifie alors le copolymère d'enzyme, ainsi obtenu, par filtration sur gel.

On active ensuite le copolymère d'enzyme en ajoutant un réactif homo ou hétérobifonctionnel dans des proportions de 5-50 moles/mole d'enzyme, à un pH compris entre 6,5 et 8. On purifie alors les copolymères d'enzyme activés par dessalage sur gel.

On effectue ensuite la conjugaison du copolymère d'enzyme activé avec la substance à activité immunologique en utilisant des proportions molaires aux environs de 10/1 à 1/10 (unité enzymatique/unité de substance à activité immunologique), de préférence 3/1 à 1/3, et de préférence encore 1/1, cette réaction s'effectuant à pH neutre.

Le procédé de préparation selon l'invention est décrit de façon plus détaillée ci-dessous pour une copolymérisation effectuée en deux étapes:

L'enzyme choisie pour effectuer la préparation du conjugué est, préalablement à la réaction de copolymérisation, traitée avec un réactif hétérobifonctionnel oxydatif des groupements carbohydrates, tel que le periodate (plusieurs centaines de moles/mole d'enzyme) à un pH compris entre 4 et 8. On élimine ensuite le periodate en excès par dialyse de la solution d'enzyme oxydée.

La solution d'enzyme oxydée est ajustée à 5-10 mg/ml et divisée en 2 parties égales.

On ajoute alors à la première partie un réactif hétérobifonctionnel tel que la 2-mercaptoéthylamine à une concentration de 1/1-10 équivalents molaires (unité enzymatique/unité de diamine), de préférence de 1/4-6 équivalents molaires, en milieu dont le pH est compris entre 7 et 9,5, et on fait réagir pendant une heure à température ambiante.

On ajoute à la deuxième partie un réactif tel que le 4-(N-maléimidométhyl)cyclohexane-1-carboxyl hydrazide à une concentration telle que définie précédemment et on fait réagir pendant une heure à température ambiante.

Selon une variante préférée, pour l'étape décrite précédemment, on effectue le protocole suivant:

- On ajoute à la première partie un réactif tel que le 3-(2-pyridyl-dithio) propionyl hydrazide (PDPH) à une concentration finale de 5 mM environ, à pH 5-7. On laisse réagir les réactifs pendant une heure environ à 20°C.
- On ajoute à la deuxième partie un réactif tel que le 4-(4-N-maléimido-phényl) butyryl hydrazide (MPBH) à une concentration finale de 1 mM environ, à pH 5-7. On laisse réagir les réactifs pendant 1 heure environ à 20°C.

Chacune des solutions d'enzyme est filtrée et purifiée par filtration sur gel.

La fraction activée par la PDPH est réduite par du dithiothréitol (concentration finale 10 mM) pendant 10 minutes environ, puis purifiée à nouveau par filtration sur gel.

La fraction d'enzyme activée par la PDPH après réduction est mélangée à la fraction d'enzyme activée à la MPBH. La réaction de copolymérisation des 2 espèces est effectuée à 20°C à un pH compris entre 6 et 8.

Selon le procédé de l'invention, le contrôle de la copolymérisation est effectué par filtration sur gel, à l'aide d'une colonne analytique, dont les caractéristiques exactes sont choisies en fonction de l'enzyme qui forme le copolymère. On utilise, par exemple, une colonne remplie de gel Pharmacia Superose® 6 Prep Grad pour la phosphatase alcaline (PM = 140000) ou de Superose® 12 Prep Grad pour la peroxydase de raifort (PM = 44000).

Selon le procédé de l'invention, à la fin de la copolymérisation, le blocage des réactions est réalisé par additions successives de 2-mercaptoéthanol (concentration finale 1 mM) et de N-éthylmaléimide (concentration finale 2 mM). Une réduction au borohydrure de sodium ou au cyanoborohydrure de sodium peut être effectuée pour réduire les fonctions carbonyles qui n'ont éventuellement pas réagi et pour stabiliser les fonctions hydrazones créées par la réaction des fonctions carbonyles sur les hydrazides.

La solution d'enzyme polymérisée est purifiée par gel filtration sur colonne préparative dans des conditions similaires au contrôle analytique précédent. La fraction correspondant aux espèces exclues du gel est récupérée et constitue la solution d'enzyme polymérisée. La concentration en protéine est déterminée par dosage BCA (Pierce).

On active ensuite le copolymère d'enzyme en ajoutant un réactif homo ou hétérobifonctionnel dans des proportions de 5-50 moles/mole d'enzyme, à pH compris entre 6,5 et 8. On purifie alors les copolymères d'enzyme activés par dessalage sur gel . On effectue alors la conjuguaison du copolymère d'enzyme activé avec la substance à activité immunologique, en utilisant des proportions molaires aux environs de 10/1 à 1/10 (unité enzymatique/unité de substance à activité immunologique), de préférence 3/1à 1/3, et de préférence encore 1/1, cette réaction s'effectuant à pH neutre.

Selon le choix du conjugué que l'on préférera obtenir, on bloquera ou non avant la réaction de copolymérisation, les fonctions amines libres des enzymes.

Dans la mesure où on ne bloque pas les fonctions amines libres des molécules d'enzyme, on utilisera de préférence au cours de la réaction de copolymérisation une diamine aromatique telle que par exemple le phénylène-1,4-diamine ou des réactifs hétérobifonfonctionnels.

Dans la mesure où on bloque les fonctions amines libres des molécules d'enzyme avant la réaction de copolymérisation avec un agent de protection des fonctions aminées, la réaction de copolymérisation peut être effectuée avec une diamine aliphatique (linéaire, ramifiée ou cyclisée) ou une amine aromatique comprenant de 2 à 12 atomes de carbone ou des réactifs hétérobifonctionnels.

On peut alors entreprendre la réaction de copolymérisation, et selon l'endroit où l'on veut que réagisse le couplage de la substance à activité immunologique par l'intermédiaire de réactif homo ou hétérobifonctionnel sur le copolymère d'enzyme, on éliminera ou non le groupement protecteur des fonctions amines.

Le couplage du copolymère d'enzyme avec la substance à activité immunologique peut s'effectuer soit sur la partie protéique du copolymère d'enzyme, soit sur les diamines ou réactifs hétérobifonctionnels ayant réagi préalablement par l'une de leurs extrémités sur les parties sucrées oxydées et n'ayant pas réagi par l'autre extrémité, soit sur les deux à la fois.

Il ressort de la description précédente que le procédé de préparation de conjugués selon l'invention, comprenant une copolymérisation en une étape, permet, en plus de l'obtention de conjugués présentant un gain considérable de sensibilité dans des tests immunoenzymatiques, des variantes intéressantes de préparation.

Il ressort également de la description précédente que l'avantage du procédé de préparation de conjugués selon l'invention comprenant une copolymérisation en deux étapes, est un éventail plus élevé de choix de sites de couplage de la substance à activité immunologique.

On peut donc obtenir par le choix du procédé de préparation des conjugués selon l'invention, en fonction de l'infection que l'on veut diagnostiquer, des immuno-conjugués appropriés constitués du copolymère d'enzyme choisi et de substance à activité immunologique spécifique de l'infection. Les combinaisons possibles du procédé de préparation des conjugués selon l'invention (que ce soit l'étape de copolymérisation d'une ou plusieurs enzymes différentes copolymérisées ou l'étape de couplage d'une ou plusieurs substances à activité immunologique utilisées) sont variées et permettent par un choix judicieux d'obtenir des conjugués qui procurent des résultats de détection spécifiques et ultrasensibles.

Dans la description des exemples qui suivent, donnés à titre non limitatif, les colonnes du système de chromatographic (gel Superpose® 12 Prep Grad et colonnes HR 10x30 et XK 16x70, PHARMACIA ) sont rincées et équilibrées avec le tampon PBS ( 50 mM / pH = 7,4 ) préalablement dégazé.

### Exemple 1: CONJUGUE DE PEROXYDASE -PEPTIDE VIH1

a- Copolymérisation de la peroxydase en une étape:

Pour réaliser la copolymérisation, on prépare les tampons (Tpn) suivants :

- Tpn PBS (phosphate de sodium 50 mM,NaCl 0,15 M / pH = 7,4)    2,0 l
- Tpn Hépès (0,36 M / pH = 5,4)    0,1 l
- Tpn Acétate de sodium (0,01 M / pH = 5,4)    au moins 1000 fois le volume de peroxydase oxydée
- Tpn Carbonate de sodium (1 M / pH= 9,0)    15% du volume de peroxydase

Pour les calculs de concentrations et quantités, les lectures de densité optique (D.O). sont effectuées à 280 et 403 nm, les coefficients d'extinction massique sont:

$$\varepsilon_{280} = 0{,}7 \text{ ml.mg}^{-1}.\text{cm}^{-1}$$
$$\varepsilon_{403} = 1{,}93 \text{ ml.mg}^{-1}.\text{cm}^{-1}$$

Le poids moléculaire de la peroxydase est de 44 000 Da.

La peroxydase (100 mg) est préparée à une concentration théorique de 45 mg/ml en tampon Hépès, puis contrôlée par une mesure de la D.O. à $\lambda$280 et 403 nm. La concentration doit être de 35 mg/ml à 15 % près. Elle est ensuite oxydée par addition de periodate (1,5 fois la quantité de peroxydase à une concentration de 100 mg/ml en tampon Hépès). Cette oxydation dure 45 minutes plus ou moins 10 minutes à environ 20°C et elle est effectuée sous agitation constante.

On procède ensuite à une dialyse qui est effectuée à 4° C en trois étapes correspondant à trois volumes de tampon acétate de sodium largement supérieurs au volume de peroxydase oxydée. La durée totale de la dialyse doit être de 24 heures environ.

La copolymérisation a lieu en milieu alcalin obtenu par addition de tampon carbonate de sodium et en présence de 4 équivalents molaires de phénylène-1,4-diamine à 2 mg/ml en tampon carbonate de sodium. Cette étape se fait à 20°C, sous agitation, et durant à peu près 12 heures.

Les contrôles de la copolymérisation sont effectués par injection de 25 μl de peroxydase copolymérisée sur gel Superose® en colonne HR 10×30 analytique. Le profil doit être composé d'un pic majoritaire correspondant à des espèces moléculaires exclues du gel. Deux ou trois autres pics peuvent être présents et correspondent aux différents états de copolymérisation. On bloque alors la réaction.

L'arrêt de la copolymérisation est rcalisée par la réduction des fonctions oxydées des molécules de peroxydase qui n' ont pas réagi pendant la copolymérisation. L'arrêt se fait par addition de borohydrure de sodium (5 % du volume de peroxydase) à 5 mg/ml en solution dans l'eau. Le mélange est maintenu sous agitation pendant 30 secondes, puis 15 à 20 minutes à température ambiante sans agitation.

On répète la même étape une seconde fois dans les mêmes conditions.

On purifie les copolymères sur gel Superose® en colonne XK 16x70 préparative. La quantité maximale injectable de peroxydase est de 200 mg, le volume maximal est de 10 ml. La colonne doit être équilibrée en tampon PBS. Le débit pour la purification est de 120 ml/heure

On collecte la fraction correspondant aux espèces moléculaires exclues ou peu retenues par le gel. Le coefficient d'extinction massique (E) pour le copolymère de peroxydase est de 1,3 ml.mg$^{-1}$.cm$^{-1}$ à $\lambda$ 403 nm.

b- Couplage:

On prépare les tampons suivants :

- Tpn PBS (phosphate de sodium 50 mM NaCl 0,15 M / pH = 7,4)          2 l
- Tpn M.B.U.:-acide 2-(N-morpholino) éthanesulphonique (MES) 5mM

  - acide borique 5mM
  - urée 2M
    pH= 7,0          2 l

Le peptide utilisé est le fragment (584-609) de la protéine gp 41 de l'isolat BRU VIH1. Ce peptide correspond au peptide 39 divulgué dans le brevet européen de Genetic Systems Corporation, dont le numéro de publication est EP-220 273.

La quantité de copolymère à activer est fonction de la quantité de peptide à coupler: 10 à 20 mg de peroxydase pour 1 mg de peptide VIH1.

Le copolymère de la peroxydase est activé avec 35 équivalents molaires de BS$^3$ à 30 mg/ml en tampon PBS pendant 45 minutes à 20°C sous agitation constante.

Le dessalage du copolymère d'enzyme activé se fait sur gel Superose® équilibré en M.B.U. et a pour rôle de séparer le copolymère de peroxydase activé du BS$^3$ n' ayant pas réagi.

Le copolymère de peroxydase est récupéré à la sortie de ce dessalage et ajusté à une concentration de 6 mg/ml.

Pour la conjugaison du copolymère de peroxydase activé et du peptide VIH1, le ratio molaire (peptide/peroxydase) est de 1/1. Le peptide est en solution à 5 mg/ml. On mélange les quantités correctes de copolymères de peroxydase activés et de peptides et on laisse sous agitation constante pendant 2 heures à 20° C.

Après deux heures de conjugaison, le mélange est purifié sur gel Superose® équilibré en tampon PBS. Le mélange copolymère de peroxydase - peptide VIH1 est injecté sur la colonne à un débit de 120 ml/heure.

La fraction correspondant au conjugué est récupérée, la concentration est déterminée par lecture de D.O. à $\lambda$ 403 nm ($\epsilon$=1,3 ml.mg$^{-1}$.cm$^{-1}$).

**Exemple 2: CONJUGUE DE PHOSPHATASE ALCALINE-PEPTIDE VIH1**

a- Copolymérisation de la phosphatase alcaline en une étape:

On effectue le même protocole de préparation que celui décrit dans l'exemple 1, mais on utilise la phosphatase alcaline purifiée à la place de la peroxydase. Le poids moléculaire de la phosphatase alcaline est de 140000 Da. Pour préparer le conjugué de l'exemple 2, la polymérisation de la phosphatase alcaline s'effectue à partir d'une solution d'enzyme à 12 mg/ml en tampon triéthanolamine (pH=7,6) contenant du NaCl 3M, du MgCl$_2$ 5mM et du ZnCl$_2$ 0,2 mM.

L'oxydation est réalisée par 400 équivalents molaires de periodate de sodium pour un équivalent molaire de phosphatase alcaline.

Les contrôles de la copolymérisation sont effectués sur gel Superose 6 Pg (Pharmacia).

Le coefficient d'extinction massique ($\epsilon$) pour le copolymère de phosphatase alcaline est de 1.4ml.mg$^{-1}$.cm$^{-1}$, à $\lambda$ 280nm au lieu de 1,0ml.mg$^{-1}$.cm$^{-1}$ pour la phosphatase non polymerisée.

b- Couplage:

Le couplage du peptide à la phosphatase alcaline polymérisée est réalisé en suivant la méthode décrite dans l'exemple 1 en changeant le copolymère d'enzyme. Après réaction de couplage, réalisé en utilisant 3 moles de peptide par mole d'unité enzymatique, le conjugué est purifié sur gel Superose® 6 PG équilibré en tampon tris 10 mM, pH 8 contenant du MgCl$_2$ (1mM) et du ZnCl$_2$ (0,1 mM). La fraction exclue du gel et qui correspond au conjugué est récupérée. La concentration est déterminée par la lecture de la densité optique à 280 nm ($\epsilon$ = 1,4ml.mg$^{-1}$.cm$^{-1}$).

MISE EN OEUVRE DU CONJUGUE PREPARE (CONJUGUE B) ET COMPARAISON AVEC UN CONJUGUE SELON L'ART ANTERIEUR (CONJUGUE A) A ENZYME NON POLYMERISEE

Pour le conjugué A, préparé selon l'art antérieur, l'enzyme utilisée n'est pas polymérisée. La méthode de couplage du peptide à l'enzyme est identique à la méthode de couplage décrite dans cet exemple.

Principe de l'immunoessai réalisé dans l'appareil Access® (Sanofi Diagnostics Pasteur)

La détection des anticorps dirigés contre le virus VIH1 est basée sur le principe de la technique immunoenzymatique chimioluminescent de type sandwich. Le test repose sur l'utilisation d'une phase solide recouverte avec des antigènes purifiés, dont la glycoprotéine d'enveloppe VIH1.

La phase solide est constituée de microbilles paramagnétiques (Estapor®, Prolabo, France) à raison de 50 µg dans 0,1 ml de diluant.

Le substrat de l'enzyme utilisé est un dioxétane de type AMPPD (Tropix) ou équivalent.

Les sérums étudiés comprennent:

- 13 échantillons positifs en anticorps anti-VIH1 (échantillons de séroconversion, dilués ou non, obtenus de BBI, NABI, Serologicals, USA).
- 44 échantillons négatifs en anticorps anti-VIH1.

Pour la mise en oeuvre du conjugué décrit dans cet exemple, on effectue les étapes suivantes:

On prépare une série de tubes étiquetés A et une série de tubes étiquetés B.

On distribue par tube:

- 0,1 ml de suspension de microbilles sensibilisées
- 0,1 ml de sérum à étudier

Après incubation de 20 minutes à 37°C et lavage (triple), on ajoute 0,26 ml de conjugué:

- tubes A: conjugué A
- tubes B: conjugué B

On met à incuber tous les tubes 20 minutes à 37°C et procède à un lavage (triple), puis on ajoute à chaque tube 0,2 ml de substrat, on laisse incuber 5 minutes à 37°C, puis on mesure dans chacun des tubes A et B , à l'aide du photomultiplicateur Access®, la lumière émise, exprimée en UAL (Unités Arbitraires de Lumière).

Résultats:

**1-**Etablissement des valeurs-seuils:

On calcule la valeur moyenne $\bar{x}$ de la lumière émise (UAL) dans tous les échantillons négatifs des tubes A et on ajoute 10 écart-types (ET), et l'on obtient la valeur $\bar{x}$A + 10 ET que l'on utilise comme valeur seuil des tubes A.

On calcule la valeur moyenne $\bar{x}$ de la lumière émise (UAL) dans tous les échantillons négatifs des tubes B et on ajoute 10 écart-types (ET), et l'on obtient la valeur $\bar{x}$B + 10ET (exprimée en UAL) que l'on utilise comme valeur seuil des tubes B.

**2-**Interprétation des résultats:

Est déclaré positif un échantillon dont la valeur de lumière émise est supérieure à la valeur seuil qui lui correspond.

Est déclaré négatif un échantillon dont la valeur de lumière émise est inférieure à la valeur seuil qui lui correspond.

Le tableau I ci-après donne les valeurs obtenues.

## TABLEAU I

| SERUMS | Conjugué A | | Conjugué B | |
|---|---|---|---|---|
| | UAL | UAL X + 10ET | UAL | UAL X + 10ET |
| S21–1/50 | 445617 | 2.91 | 1339800 | 15.16 |
| S16–1/3 | 179289 | 1.17 | 537851 | 6.08 |
| S14–1/1 | 150627 | 0.98 | 466845 | 5.28 |
| BBI Q4–1/40 | 129214 | 0.84 | 102312 | 1.16 |
| BBI Q6–1/10 | 513387 | 3.36 | 962563 | 10.89 |
| BBI Q6–1/100 | 128650 | 0.84 | 144469 | 1.63 |
| BBI Q6–1/200 | 106540 | 0.70 | 106991 | 1.21 |
| BBI 18 1/1 | 211733 | 1.38 | 428775 | 4.85 |
| NABI 241 C | 88785 | 0.58 | 83407 | 0.94 |
| NABI 241 D 1/10 | 206477 | 1.35 | 599059 | 6.78 |
| NABI 241 D 1/50 | 154701 | 1.01 | 380851 | 4.31 |
| NABI 241 D 1/100 | 123333 | 0.81 | 224706 | 2.54 |
| BBI K5 1/10 | 139337 | 0.91 | 181874 | 2.06 |
| $\bar{X}$ n = 44 | 86809 | | 45719 | |
| SD | 6614 | | 4268 | |
| $\bar{X}$ + 10SD (=VS)* | 152947 | | 88398 | |

\* VS = Valeur Seuil

Il ressort très clairement des résultats exposés que le conjugué selon l'invention (B) permet d'obtenir des réponses UAL très significativement augmentées par rapport à celles du conjugué A. En outre, on remarquera que six sérums (N°514; Q4-1/40: Q6-1/100; Q6-1/200; 241D 1/100: K5 1/10) trouvés négatifs avec le conjugué de l'art antérieur (A) deviennent positifs avec le conjugué selon l'invention (B).
Ces essais montrent donc bien que l'invention permet d'obtenir une sensibilité améliorée.

## Exemple 4: CONJUGUE DE PHOSPHATASE ALCALINE-PEPTIDE VIH1

a- Copolymérisation de la phosphatase alcaline en deux étapes:

La phosphatase alcaline en solution (10-20mg/ml dans un tampon triéthanolamine 30 mM, pH 7,6 contenant du NaCl 3 M, du $MgCl_2$ 1 mM et du $ZnCl_2$ 0,1 mM) est oxydée par le périodate de sodium (300 moles/mole d'enzyme) pendant 45 minutes à 20°C.
On élimine ensuite le periodate en excès par dialyse de la solution d'enzyme oxydée contre du tampon acétate de sodium 10mM pH 5,5 contenant 1mm de $MgCl_2$
Après dialyse, la solution d'enzyme oxydée est divisée en 2 parties égales.
On ajoute à la première partie le 3-(2-pyridyl-dithio) propionyl hydrazide (PDPH) à une concentration finale de 4 mM, la concentration de la phosphatase alcaline étant ajustée à 10 mg/ml. La réaction dure 1 heure sous agitation à 20°C.
On ajoute à la deuxième partie le 4-(4N-malémido-phényl)butyryl hydrazide (MPBH) à une concentration

9

finale de 1 mM, la concentration de la phosphatase alcaline étant ajustée à 5 mg/ml. La réaction dure 1 heure sous agitation à 20°C.

Chacune des solutions d'enzyme est filtrée et purifiée par filtration sur gel en tampon phosphate de sodium 50 mM, pH 7,4, contenant 150 mM de chlorure de sodium.

La fraction activée par la PDPH est réduite par du dithiothréitol (concentration finale 10 mM) pendant 10 minutes puis purifiée à nouveau par filtration sur gel.

La fraction d'enzyme activée par le PDPH après réduction est mélangée à la fraction d'enzyme activée au MPBH. La réaction de copolymérisation des deux espèces est effectuée à 20°C sous agitation pendant 2 à 15 heures selon la taille des copolymères que l'on désire obtenir.

Le contrôle de la copolymérisation est effectué par filtration sur gel sur une colonne analytique remplie de gel Superose® 6 Prep Grad (Pharmacia).

Le blocage des réactions est réalisé par additions successives de 2-mercaptoéthanol (concentration finale 1 mM) et de N-éthylmaléimide (concentration finale 2 mM). Une réduction au borohydrure de sodium ou au cyanoborohydrure de sodium peut être effectuée pour réduire les fonctions carbonyles qui n'ont éventuellement pas réagi et pour stabiliser les fonctions hydrazones créées par la réaction des fonctions carbonyles sur les hydrazides.

La solution d'enzyme polymérisée est purifiée par gel filtration sur colonne préparative dans des conditions similaires à celles du contrôle analytique précédent. La fraction correspondant aux espèces exclues du gel est récupérée et constitue la solution d'enzyme polymérisée. La concentration en protéine est déterminée par dosage BCA (Pierce).

b- Couplage:

On effectue le même protocole que celui décrit dans l'exemple 2.

**Exemple 6: IMMUNOENZYMOESSAI DU CONJUGUE PEROXYDASE-PEPTIDE VHC (ou HCV)**

La détection des anticorps dirigés contre le virus de l'hépatite C (HCV ou VHC) est basée dans l'exemple ci-après sur le principe de la technique immunoenzymatique de type sandwich. Le test repose sur l'utilisation d'une phase solide (microplaque) sensibilisée avec un antigène purifié du virus VHC (capside).

La mise en oeuvre du test repose sur les étapes réactionnelles suivantes.

Chaque sérum à étudier (0,1 ml d'échantillon dilué au 3/4) est distribué dans une cupule de la microplaque. Puis, le conjugué (0,1ml) marqué à la péroxydase est ajouté. Après incubation 30 minutes à 40°C, puis lavage, la présence de l'enzyme immobilisée sur les complexes est révélée par incubation en présence du substrat (TMB) pendant 30 minutes. Après arrêt de la réaction avec du $H_2SO_4$, la lecture s'effectue au spectrophotomètre à λ450/620 nm. La présence ou l'absence des anticorps anti-VHC est déterminée en comparant pour chaque échantillon la densité optique (DO) enregistrée à celle de la valeur-seuil calculée (dans ce cas, moyenne des négatifs + 0,2).

Le tableau IV montre les résultas comparatifs obtenus à l'aide de 2 conjugués, A et B, fabriqués dans des conditions différentes (couplage d'un peptide mimant une partie de la protéine de capside du virus VHC).

Les conjugués A et B préparés, sont décrits ci-après:

- Conjugué A: la peroxydase est non traitée et le peptide est couplé à l'aide d'un réactif bifonctionnel (PBS). La méthode de couplage est celle décrite dans l'exemple 1 en utilisant la peroxydase.

- Conjugué B: le conjugué est préparé suivant la méthode décrite dans l'exemple 1 en remplaçant le peptide VIH par le peptide VHC.

## TABLEAU IV

| Echantillons | Conjugué A DO | Conjugué B DO |
|---|---|---|
| Sérum négatif | 0.012 | 0.025 |
| Sérums Positifs | | |
| PHV – 903 – 1 | 0.109 | 1.398 |
| PHV – 903 – 2 | 0.308 | > 3,0 |
| PHV – 903 – 3 | 0.300 | > 3,0 |
| PHV – 903 – 4 | 0.817 | > 3,0 |
| PHV – 903 – 5 | 0.555 | > 3,0 |
| PHV – 903 – 6 | 0.448 | > 3,0 |

Le tableau IV montre l'augmentation de sensibilité obtenue sur tous les prélèvements de la séroconversion PHV-903- (Panel de Boston Biomedica Inc.). Le premier prélèvement détecté par les tests actuels, connus, se situe entre le n° 2 et le n° 6, mais n'est jamais le n°1.

Ces résultats confirment les résultats précédents et montrent l'intérêt de la présente invention.

### Exemple 7: IMMUNOENZYMOESSAI METTANT EN OEUVRE UN CONJUGUE PEROXYDASE-PEPTIDE VIH1

La détection des anticorps dirigés contre le virus VIH est basée dans l'exemple ci-après sur le principe de la technique immunoenzymatique de type sandwich. Le test repose sur l'utilisation d'une phase solide préparée avec des antigènes purifiés, dont la glycoprotéine d'enveloppe du virus VIH1.

Les sérums à étudier (0,1 ml d'échantillon dilué au 3/4) sont distribués dans les cupules de la microplaque. Après incubation de 30 minutes, le conjugué marqué à la peroxydase est ajouté après lavage. La présence de l'enzyme immobilisée sur les complexes est révélée par incubation en présence du substrat après élimination de la fraction de conjugué restée libre. Après arrêt de la réaction, la lecture s'effectue au spectrophotomètre à 450/620 nm. La présence ou l'absence d'anticorps anti-VIHI est déterminée en comparant pour chaque échantillon la densité optique enregistrée à celle de la valeur seuil calculée (dans ce cas, moyenne des négatifs + 0,1).

Le tableau IV montre les résultats comparatifs obtenus à l'aide de cinq conjugués fabriqués dans des conditions différentes (couplage d'un peptide mimant l'épitope immunodominant de la glycoprotéine d'enveloppe du virus VIH1/acides aminés 584-609, isolat BRU, à la peroxydase).

Les différents conjugués préparés sont décrits ci après:

- Conjugué A: la peroxydase est non traitée et le peptide est couplé à l'aide d'un réactif bifonctionnel (la méthode de couplage est celle de l'exemple 1).

- Conjugué B: le peptide est couplé à la peroxydase suivant la méthode Nakane et al (J.Histochem.Cytochem, 1974,22,1084-1091) modifiée de la façon suivante: 28 mg de peroxydase sont oxydés avec 19,7 mg de periodate de sodium pendant 1 heure 30 minutes. 1 mg de peptide est mis en contact avec 4 mg de peroxydase oxydée pendant 3 heures. Ce protocole ne modifie pas le couplage en tant que tel selon la méthode Nakane.

- Conjugué C: la peroxydase est préparée suivant la méthode décrite dans la demande de brevet publiée sous le numéro EP 601318, et couplée au peptide à l'aide d'un réactif bifonctionnel selon la méthode décrite dans le procédé de ladite demande.

- Conjugué D: le conjugué est préparé suivant la méthode décrite dans l'exemple 1, en utilisant l'hexanediamine-1,6 comme réactif bifonctionnel de copolymérisation.

- Conjugué E: le conjugué est préparé suivant la méthode décrite dans l'exemple 1.

Dans les tableaux qui suivent, les résultats donnés sont des valeurs de densité optique lues en effectuant des essais immunoenzymatiques suivant le protocole décrit et en utilisant les conjugués A,B,C,D et E décrits.

Les sérums utilisés sont des échantillons des panels K, U et Q fournis par Boston Biomedica Inc., ainsi que 31 sérums de patients donneurs normaux et négatifs pour la détection d'anticorps VIH.

|  | A | B | C | D | E |
|---|---|---|---|---|---|
| Panel K | DO | DO | DO | DO | DO |
| K1 | 0,047 | 0,053 | 0,062 | 0,046 | 0,122 |
| K2 | 0,095 | 0,161 | 0,106 | 0,835 | 1,651 |
| K3 | 0,195 | 0,310 | 0,161 | 2,106 | 3 |
| K4 | 0,243 | 0,463 | 0,399 | 2,816 | 3 |

| Panel U |  |  |  |  |  |
|---|---|---|---|---|---|
| U1 | 0,045 | 0,112 | 0.298 | 2,407 | 3 |
| U2 | 0,226 | 1,687 | 3 | 3 | 3 |

| Panel Q |  |  |  |  |  |
|---|---|---|---|---|---|
| Q3 | 0,050 | 0,044 | 0,049 | 0,055 | 0,125 |
| Q4 | 0,066 | 0,368 | 1,052 | 2,816 | 3 |
| Q5 | 0,125 | 0,404 | 1,064 | 2,906 | 3 |
| Q6 | 0,116 | 0,382 | 1,009 | 2,836 | 3 |
| Q6 1/2 | 0,100 | 0,241 | 0,743 | 2,191 | 3 |
| Q6 1/4 | 0,073 | 0,208 | 0,569 | 2,133 | 3 |
| Q6 1/8 | 0,055 | 0,135 | 0,356 | 1,523 | 2,837 |
| Q6 1/16 | 0,047 | 0,092 | 0,218 | 0,965 | 1,945 |
| Q6 1/32 | 0,047 | 0,081 | 0,163 | 0,668 | 1,515 |
| Q6 1/64 | 0,042 | 0,064 | 0,118 | 0,430 | 0,772 |

| moyenne (n=31) | 0,039 | 0,047 | 0,040 | 0,031 | 0,037 |
|---|---|---|---|---|---|
| valeur seuil (vs) | 0,139 | 0,147 | 0,140 | 0,131 | 0,137 |

Les résultats des tests rassemblés dans les tableaux montrent que seuls les conjugués D et E permettent de détecter la positivité à la fois des sérums K2 et Q6 dilués au 1/64.

Il apparait donc que les conjugués selon l'invention permettent d'obtenir une sensibilité de détection meilleure que ceux connus de l'art antérieur. La détection des anticorps dirigés contre les protéines du virus VIH1 est ainsi plus précoce.

**Revendications**

1.  Conjugué immunoenzymatique d'enzymes de marquage glycosylées et de substances à activité immunologique, caractérisé en ce qu'il est constitué

    -   des molécules d'enzyme de marquage copolymérisées entre elles, par l'intermédiaire de leurs groupes glucidiques préalablement oxydés, de manière à former un copolymère d'enzyme
    -   et d'au moins une substance à activité immunologique conjuguée aux molécules d'enzyme de marquage copolymerisée par l'intermédiaire de fonctions amines libres du copolymère d'enzyme.

2.  Conjugué selon la revendication 1, caractérisé en ce que le copolymère d'enzyme est obtenu à partir d'enzyme de marquage et de diamine(s), ou d'enzyme de marquage et de réactifs hétérobifonctionnels différents, liés entre eux.

3.  Conjugué selon la revendication 2, caractérisé en ce que la diamine est choisie parmi une diamine comprenant 2 à 12 atomes de carbone, aliphatique à chaîne linéaire ou ramifiée ou cyclisée ou une diamine aromatique.

4.  Conjugué selon la revendication 2, caractérisé en ce que la diamine est le phénylène-1,4-diamine.

5.  Conjugué selon la revendication 2, caractérisé en ce que les réactifs hétérobifonctionnels différents sont deux réactifs hétérobifonctionnels respectivement choisis parmi la 2-mercapto-éthylamine ou le 3-( 2-pyridyldithio) propionyl hydrazide et le 4-(N-maléimidométhyl) cyclohexane-1-carboxyl hydrazide ou le 4-(4-N-maléimidophényl) butyryl hydrazide.

6.  Conjugué selon la revendication 2. caractérisé en ce que les proportions d'enzyme et de diamine ou de réactifs hétérobifonctionnels sont respectivement de 1/1-10 équivalents molaires, et de préférence 1/4-6 équivalents molaires.

7.  Conjugué selon la revendication 1, caractérisé en ce que le copolymère d'enzyme comprend n molécules d'enzyme, n étant un nombre entier compris entre 3 et 100, de préférence un nombre entier de 5 à 50.

8.  Conjugué selon les revendications 1 à 7, caractérisé en ce que l'enzyme copolymérisée est la peroxydase de raifort ou la phosphatase alcaline.

9.  Conjugué selon la revendication 1, caractérisé en ce que le copolymère d'enzyme est couplé à au moins une substance à activité immunologique par l'intermédiaire d'un réactif homo ou hétérobifonctionnel.

10. Conjugué selon les revendications 1 et 9, caractérisé en ce que les proportions molaires respectives du copolymère d'enzyme et de la substance à activité immunologique sont de 10/1 à 1/10 (unité enzymatique/unité de substance à activité immunologique), de préférence 3/1 à 1/3, et de préférence encore 1/1.

11. Conjugué selon les revendications 1,9 et 10, caractérisé en ce que la substance à activité immunologique est un peptide VIH1, un peptide VIH2, un peptide VHC ou un anticorps monoclonal-anti-VIH1 ou un anticorps anti AgHBs.

12. Procédé de préparation de conjugué selon la revendication 1, caractérisé en ce que:

    a. on copolymérise les molécules d'enzyme de marquage par l'intermédiaire de leurs carbohydrates préalablement oxydés,

    b. puis on effectue le couplage du copolymère d'enzyme avec au moins une substance à activité immunologique.

13. Procédé selon la revendication 12, caractérisée en ce que pour effectuer la copolymérisation on fait réagir les molécules d'enzyme avec une diamine.

**14.** Procédé selon la revendication 12, caractérisé en ce que pour effectuer la copolymérisation, on fait réagir séparément dans une première étape les molécules d'enzyme avec deux réactifs hétérobifonctionnels différents, puis dans une deuxième étape on fait réagir entre eux les produits de réaction.

**15.** Procédé selon les revendications 12 et 13, caractérisé en ce que après la copolymérisation, on effectue une réduction avec un agent réducteur choisi parmi le borohydrure de sodium et le cyanoborohydrure de sodium.

**16.** Procédé selon les revendications 12 à 14, caractérisé en ce que après la copolymérisation, on effectue une réaction avec des agents bloquants des réactifs hétérobifonctionnels.

**17.** Procédé selon la revendication 12, caractérisé en ce que dans l'étape du couplage du copolymère d'enzyme avec la ou les substance(s) à activité immunologique, la concentration du réactif homo ou hétérobifonctionnel est en excès par rapport à la concentration du copolymère d'enzyme.

**18.** Utilisation du conjugué selon la revendication 1 pour la détermination immunologique.

**19.** Trousse de diagnostic pour la détermination immunologique, caractérisé en ce qu'il comprend un conjugué immunoenzymatique selon la revendication 1.

**Patentansprüche**

**1.** Immunoenzymatisches Konjugat aus glykosylierten Markierungsenzymen und Substanzen mit immunologischer Wirkung, dadurch gekennzeichnet, daß es gebildet ist aus

- Markierungsenzymmolekülen, die über zuvor oxidierte Kohlenhydratgruppen copolymerisiert sind unter Bildung eines Enzym-Copolymers und
- mindestens einer mit den Markierungsenzymmolekülen konjugierten Substanz mit immunologischer Wirkung, die über die freien Aminfunktionen des Enzym-Copolymers copolymerisiert ist.

**2.** Konjugat nach Anspruch 1, dadurch gekennzeichnet, daß das Enzym-Copolymer erhalten worden ist ausgehend von dem Markierungsenzym und Diamin(en) oder dem Markierungsenzym und verschiedenen heterobifunktionellen Reagenzien, die untereinander verbunden sind.

**3.** Konjugat nach Anspruch 2, dadurch gekennzeichnet, daß das Diamin ausgewählt ist aus aliphatischen Diaminen mit 2 bis 12 Kohlenstoffatomen mit gerader oder verzweigter oder cyclisierter Kette oder aromatischen Diaminen.

**4.** Konjugat nach Anspruch 2, dadurch gekennzeichnet, daß das Diamin Phenylen-1,4-diamin ist.

**5.** Konjugat nach Anspruch 2, dadurch gekennzeichnet, daß die verschiedenen heterobifunktionellen Reagenzien zwei heterobifunktionelle Reagenzien sind, die ausgewählt sind aus 2-Mercapto-ethylamin oder 3-(2-Pyridyldithio)-propionyl-hydrazid bzw. 4-(N-Maleimidomethyl)-cyclohexan-1-carboxyl-hydrazid oder 4-(4-N-Maleimidophenyl)-butyryl-hydrazid.

**6.** Konjugat nach Anspruch 2, dadurch gekennzeichnet, daß die Verhältnisse von Enzym und Diamin oder heterobifunktionellen Reagenzien 1/1 - 10 Moläquivalente, vorzugsweise 1/4 - 6 Moläquivalente betragen.

**7.** Konjugat nach Anspruch 1, dadurch gekennzeichnet, daß das Enzym-Copolymer n Enzymmoleküle umfaßt, wobei n eine ganze Zahl zwischen 3 und 100, vorzugsweise eine ganze Zahl von 5 bis 50, bedeutet.

**8.** Konjugat nach den Ansprüchen 1 bis 7, dadurch gekennzeichnet, daß das copolymerisierte Enzym Meerrettich-Peroxidase oder alkalische Phosphatase ist.

**9.** Konjugat nach Anspruch 1, dadurch gekennzeichnet, daß das Enzym-Copolymer über ein homo- oder heterobifunktionelles Reagens an mindestens eine Substanz mit immunologischer Aktivität gekuppelt ist.

**10.** Konjugat nach den Ansprüchen 1 und 9, dadurch gekennzeichnet, daß die Molverhältnisse des Enzym-Copolymers bzw. der Substanz mit immunologischer Aktivität 10/1 bis 1/10 (enzymatische Einheit / Einheit der Substanz mit immunologischer Aktivität), vorzugsweise 3/1 bis 1/3, und noch bevorzugter 1/1 betragen.

11. Konjugat nach den Ansprüchen 1, 9 und 10, dadurch gekennzeichnet, daß die Substanz mit immunologischer Aktivität ein Peptid VIH1, ein Peptid VIH2, ein Peptid VHC oder ein monoklonaler Anti-VIH1-Antikörper oder ein Anti-AgHBs-Antikörper ist.

12. Verfahren zur Herstellung des Konjugats nach Anspruch 1, dadurch gekennzeichnet, daß man

    a. die Markierungsenzymmoleküle über ihre zuvor oxidierten Kohlenhydrate copolymerisiert,
    b. dann die Kupplung des Enzym-Copolymers mit mindestens einer Substanz mit immunologischer Aktivität bewirkt.

13. Verfahren nach Anspruch 12, dadurch gekennzeichnet, daß man zur Durchführung der Copolymerisation die Enzym-Moleküle mit einem Diamin umsetzt.

14. Verfahren nach Anspruch 12, dadurch gekennzeichnet, daß man zur Durchführung der Copolymerisation getrennt in einer ersten Stufe die Enzym-Moleküle mit zwei verschiedenen heterobifunktionellen Reagenzien umsetzt und dann in einer zweiten Stufe die Reaktionsprodukte miteinander umsetzt.

15. Verfahren nach den Ansprüchen 12 und 13, dadurch gekennzeichnet, daß man nach der Copolymerisation eine Reduktion mit einem Reduktionsmittel ausgewählt aus Natriumborhydrid und Natriumcyanoborhydrid durchführt.

16. Verfahren nach den Ansprüchen 12 bis 14, dadurch gekennzeichnet, daß man nach der Copolymerisation eine Reaktion mit Mitteln durchführt, welche die heterobifunktionellen Reagenzien blockieren.

17. Verfahren nach Anspruch 12, dadurch gekennzeichnet, daß in der Stufe der Kupplung des Enzym-Copolymers mit der oder den Substanz(en) mit immunologischer Aktivität die Konzentration des homo- oder heterobifunktionellen Reagens überschüssig ist im Vergleich zu der Konzentration des Enzym-Copolymers.

18. Verwendung des Konjugats nach Anspruch 1 für immunologische Bestimmungen.

19. Diagnose-Kit für immunologische Bestimmungen, dadurch gekennzeichnet, daß er ein immunoenzymatisches Konjugat nach Anspruch 1 umfaßt.

**Claims**

1. Immunoenzymatic conjugate of glycosylated labelling enzymes and immunologically active substances, characterised in that it consists of:

    - molecules of labelling enzyme copolymerised with one another, via their previously oxidised glucide groups, so as to form an enzyme copolymer
    - and at least one immunologically active substance conjugated with the labelling enzyme molecules, copolymerised via free amine functions of the enzyme copolymer.

2. Conjugate according to claim 1, characterised in that the enzyme copolymer is obtained from labelling enzyme and diamine(s) or labelling enzyme and different heterobifunctional reagents, bound to one another.

3. Conjugate according to claim 2, characterised in that the diamine is selected from a straight-chain, branched or cyclic aliphatic diamine having 2 to 12 carbon atoms or an aromatic diamine.

4. Conjugate according to claim 2, characterised in that the diamine is phenylene-1,4-diamine.

5. Conjugate according to claim 2, characterised in that the different heterobifunctional reagents are two heterobifunctional reagents selected from among 2-mercapto-ethylamine or 3-(2-pyridyldithio)propionyl hydrazide and 4-(N-maleimidomethyl)cyclohexane-1-carboxyl hydrazide or 4-(4-N-maleimidophenyl)butyryl hydrazide, respectively.

6. Conjugate according to claim 2, characterised in that the proportions of enzyme and diamine or heterobifunctional reagents are, respectively, 1/1-10 molar equivalents, preferably 1/4-6 molar equivalents.

7. Conjugate according to claim 1, characterised in that the enzyme copolymer comprises n molecules of enzyme, n being an integer between 3 and 100, preferably an integer from 5 to 50.

8. Conjugate according to claims 1 to 7, characterised in that the copolymerised enzyme is horseradish peroxidase or alkaline phosphatase.

9. Conjugate according to claim 1, characterised in that the enzyme copolymer is coupled to at least one immunologically active substance via a homo- or heterobifunctional reagent.

10. Conjugate according to claims 1 and 9,
characterised in that the respective molar proportions of enzyme copolymer and immunologically active substance are from 10/1 to 1/10 (enzymatic unit/unit of immunologically active substance), preferably from 3/1 to 1/3, and more preferably 1/1.

11. Conjugate according to claims 1, 9 and 10,
characterised in that the immunologically active substance is a VIH1 peptide, a VIH2 peptide, a VHC peptide or a monoclonal anti-VIH1 antibody or an anti-AgHBs antibody.

12. Process for preparing conjugate according to claim 1, characterised in that:

    a. the labelling enzyme molecules are copolymerised via their previously oxidised carbohydrates,
    b. then the enzyme copolymer is coupled to at least one immunologically active substance.

13. Process according to claim 12, characterised in that, in order to carry out copolymerisation, the enzyme molecules are reacted with a diamine.

14. Process according to claim 12, characterised in that, in order to carry out copolymerisation, in a first step the enzyme molecules are reacted separately with two different heterobifunctional reagents, then in a second step the reaction products are reacted with one another.

15. Process according to claims 12 and 13,
characterised in that, after copolymerisation, reduction is carried out with a reducing agent selected from sodium borohydride and sodium cyanoborohydride.

16. Process according to claims 12 to 14, characterised in that, after copolymerisation, a reaction is carried out with agents which block heterobifunctional reagents.

17. Process according to claim 12, characterised in that, in the step of coupling the enzyme copolymer to the immunologically active substance(s), the concentration of the homo- or heterobifunctional reagent is in an excess relative to the concentration of the enzyme copolymer.

18. Use of the conjugate according to claim 1 for immunological assay.

19. Diagnostic kit for immunological assay,
characterised in that it comprises an immunoenzymatic conjugate according to claim 1.